# EUROPÄISCHE PATENTSCHRIFT

(11) **EP 0 494 416 B1**
(45) Veröffentlichungstag und Bekanntmachung des Hinweises auf die Patenterteilung: **01.03.1995**
(21) Anmeldenummer: 91121767.7
(22) Anmeldetag: 19.12.1991
(51) Int. Cl.: C07C 51/47, C07C 51/42, C07C 55/14

(54) **Verfahren zur Aufarbeitung von Mutterlaugen, die bei der Herstellung von Adipinsäure anfallen**
Process for the treatment of waste mother liquors from the preparation of adipic acid
Procédé de traitement des liqueurs-mères de déchets à partir de préparation d'acide adipique

(30) Priorität: 10.01.1991 DE 4100505 U
(43) Veröffentlichungstag der Anmeldung: 15.07.1992
(73) Patentinhaber: BASF Aktiengesellschaft, 67063 Ludwigshafen (DE)
(72) Erfinder: Frank, Gerhard, Dr., W-6945 Hirschberg (DE); Herrmann, Guenter, Dr., W-6900 Heidelberg (DE); Buerger, Gert, Dr., W-6800 Mannheim 24 (DE); Manegold, Jost Henrich, Dr., W-6715 Lambsheim (DE); Karbach, Stefan, Dr., W-6730 Neustadt (DE); Schmitz, Ruediger, W-6715 Lambsheim (DE)

(56) Entgegenhaltungen:
- EP-A- 0 128 709
- DE-A- 1 938 103
- DE-C- 920 788
- US-A- 3 106 450
- US-A- 3 965 164

## Beschreibung

Bei der Herstellung von Adipinsäure durch Oxidation von Cyclohexanol oder Gemischen aus Cyclohexanol und Cyclohexanon in Gegenwart von Kupfer- und Vanadiumkatalysatoren mit Salpetersäure erhält man nach Abkühlen und Abscheiden der auskristallisierten Adipinsäure eine Mutterlauge, die nach Ergänzung mit Salpetersäure wiederum zurückgeführt wird. Um eine Anreicherung an Nebenprodukten zu vermeiden, wird ein Teil dieser Mutterlauge ausgeschleust und aufgearbeitet. Hierbei wird ein Gemisch aus Dicarbonsäuren, im wesentlichen Adipinsäure, Glutarsäure und Bernsteinsäure, erhalten. Ferner ist man bestrebt, die Katalysatormetalle Kupfer und Vanadium zurückzugewinnen und wieder für die Oxidation zu verwenden.

Aus der deutschen Patentschrift 920 788 ist bekannt, daß man Mutterlaugen aus der Adipinsäureherstellung nach Abscheiden der Adipinsäure unter vermindertem Druck bis zur Trockene eindampft, den erhaltenen Rückstand bis zur Beendigung der Stickoxidbildung auf eine Temperatur bis zu 200°C erhitzt und anschließend das Dicarbonsäuregemisch durch Umkristallisieren oder Destillation reinigt. Das so gewonnene Dicarbonsäuregemisch entspricht nicht mehr den gestellten Anforderungen und darüber hinaus gehen die Katalysatormetalle verloren.

Nach einem anderen in der US-Patentschrift 3 106 450 beschriebenen Verfahren dampft man die Mutterlauge aus der Adipinsäureherstellung bei einer Temperatur von 130 bis 160°C unter vermindertem Druck ein und destilliert die Salpetersäure hierbei azeotrop ab und gibt anschließend soviel Wasser zu, daß eine Lösung mit einen pH-Wert von 1,5 bis 6 entsteht. Diese Lösung wird mit einem Ionentauscher behandelt und hierbei das Katalysatormetall abgetrennt, das anschließend durch Eluieren von dem Ionentauscher wieder gewonnen werden kann. Auch dieses Verfahren ist hinsichtlich der Qualität der erhaltenen Dicarbonsäuren unbefriedigend, darüber hinaus ist die Wiedergewinnungsrate bei den Katalysatormetallen verbesserungsbedürftig.

Es war deshalb die technische Aufgabe gestellt, aus Mutterlaugen, die bei der Adipinsäureherstellung anfallen, das Dicarbonsäuregemisch mit möglichst geringem Stickstoffgehalt in hoher Ausbeute zu gewinnen, das sich mit Polyolen zu Polyesterolen mit einer niedrigen Farbzahl umsetzen läßt und zugleich die Katalysatormetalle mit einer hohen Wiedergewinnungsrate zurückgewonnen werden.

Diese Aufgabe wird gelöst in einem Verfahren zur Aufarbeitung von Mutterlaugen, die bei der Herstellung von Adipinsäure durch Oxidation von Cyclohexanol oder Cyclohexanon oder deren Gemische mit Salpetersäure in Gegenwart von Kupfer- und Vanadiumkatalysatoren nach Abtrennung der Adipinsäure erhalten wurden, welches folgende Schritte umfaßt:
a) Abdestillieren der Salpetersäure als Azeotrop mit Wasser unter vermindertem Druck unter Erhalt einer Kupfer- und Vanadiumionen enthaltenden Dicarbonsäureschmelze,
b) Erhitzen der Kupfer- und Vanadiumionen enthaltenden Dicarbonsäureschmelze auf eine Temperatur von 130 bis 180°C unter Zersetzung von Stickstoffverbindungen und Oxalsäure und Erhalt einer Kupfer- und Vanadiumionen enthaltenden Dicarbonsäureschmelze mit vermindertem Stickstoffgehalt,
c) Lösen der in b) erhaltenen Kupfer- und Vanadiumionen enthaltenden Dicarbonsäureschmelze mit vermindertem Stickstoffgehalt in Wasser unter Bildung einer wäßrigen Lösung von Dicarbonsäuren mit einem Gehalt an Kupfer und Vanadiumionen,
d) in Berührung bringen der Lösung von Dicarbonsäuren mit einem Gehalt an Kupfer- und Vanadiumionen mit einem Kationenaustauscher unter Bindung der Kupfer- und Vanadiumionen und Bildung einer im wesentlichen Kupfer- und Vanadiumionen freien Dicarbonsäurelösung,
e) Abdestillieren des Wassers aus der im wesentlichen Kupfer- und Vanadiumionen freien Dicarbonsäurelösung unter Erhalt einer Schmelze von Dicarbonsäuren,
f) Erhitzen der Schmelze von Dicarbonsäuren auf eine Temperatur von 200 bis 240°C und
g) Gewinnung eines Dicarbonsäuregemisches durch Destillation.

Das neue Verfahren hat den Vorteil, daß man das Dicarbonsäuregemisch in hoher Ausbeute erhält, wobei die Dicarbonsäuren sich durch einen niederen Stickstoffgehalt auszeichnen und Polyesterole mit einem niedrigen Farbwert ergeben. Ferner hat das neue Verfahren den Vorteil, daß eine hohe Rückgewinnungsrate für die Kupfer- und Vanadiumkatalysatoren ermöglicht wird.

Erfindungsgemäß geht man von Mutterlaugen aus, die bei der Herstellung von Adipinsäure durch Oxidation von Cyclohexanol oder Cyclohexanon oder deren Gemischen mit Salpetersäure in Gegenwart von Kupfer- und Vanadiumkatalysatoren nach Abtrennung der Mutterlauge erhalten wurden. Solche Mutterlaugen enthalten neben Verunreinigungen Salpetersäure, Wasser, Bernsteinsäure, Glutarsäure, Adipinsäure, Kupferionen, Vanadium und Eisenionen. Ein typisches Gemisch enthält beispielsweise 30 bis 36 Gew.-% Salpetersäure, 15 bis 21 Gew.-% Wasser, 10 bis 13 Gew.-% Bernsteinsäure, 23 bis 30 Gew.-% Glutarsäure, 9 bis 11 Gew.-% Adipinsäure, 0,7 bis 0,8 Gew.-% Kupfer, 0,08 bis 0,09 Gew.-% Vanadium, 0,002 bis 0,004 Gew.-% Eisen sowie weitere Verunreinigungen wie Stickstoffverbindungen.

In der Stufe a) wird aus der Mutterlauge zunächst Salpetersäure als Azeotrop mit Wasser unter vermindertem Druck unter Erhalt einer Kupfer- und Vanadiumionen enthaltenden Dicarbonsäureschmelze abdestilliert. Vorteilhaft hält man einen Druck von 50 bis 200 mbar, insbesondere 100 bis 150 mbar ein und destilliert zweckmäßig bis zu einer Temperatur von 130°C ab. Die Dicarbonsäureschmelze enthält neben Kupfer- und Vanadiumionen Verunreinigungen sowie Adipinsäure, Glutarsäure, Bernsteinsäure und Oxalsäure.

In der Stufe b) wird die Kupfer- und Vanadiumionen enthaltende Dicarbonsäureschmelze auf eine Temperatur von 130 bis 180°C erhitzt unter Zersetzung von Stickstoffverbindungen und Oxalsäure und Erhalt einer stickstoffärmeren Kupfer-und Vanadiumionen enthaltenden Dicarbonsäureschmelze. Vorteilhaft erhitzt man auf eine Temperatur von 150 bis 160°C und hält hierbei einen Druck von z.B. 150 bis 950 mbar ein. Vorzugsweise hält man die Schmelze 5 bis 60 Min. auf der vorgenannten Temperatur. Das erhaltene Gemisch hat vorteilhaft einen Gehalt an Nitrationen von weniger als 1000 mg/kg z.B. von 100 bis 500 mg je kg Dicarbonsäuregemisch.

In der Stufe c) wird die Kupfer- und Vanadiumionen enthaltende Schmelze mit vermindertem Stickstoffgehalt in Wasser gelöst unter Bildung einer wäßrigen Lösung enthaltend Dicarbonsäuren und Kupfer- und Vanadiumionen. Vorteilhaft erhält man eine 50 bis 65 gew.-%ige Dicarbonsäurelösung, die zweckmäßig für einen Zeitraum von 30 bis 60 Min. auf eine Temperatur von 70 bis 90°C gehalten wird.

In der Stufe d) wird dann die Kupfer- und Vanadiumionen enthaltende wäßrige Lösung von Dicarbonsäuren mit erniedrigtem Stickstoffgehalt mit einem Kationenaustauscher in Berührung gebracht und die Kupfer- und Vanadiumionen gebunden und eine im wesentlichen kupfer- und vanadiumfreie Dicarbonsäurelösung erhalten. Zweckmäßig leitet man die wäßrige Dicarbonsäurelösung über ein Bett von Kationenaustauschern, wie Sulfonsäuregruppen enthaltendes Polystyrol. Sobald die Austauscherschicht erschöpft ist, werden Kupfer und Vanadium durch Eluieren mit Salpetersäure wiedergewonnen. Überraschenderweise werden Eisenionen nach Durchführung der Maßnahme b) von Ionentauscher nicht festgehalten, sondern mit der Dicarbonsäurelösung ausgetragen.

Aus der in Stufe d) erhaltenen wesentlichen kupfer- und vanadiumfreien Dicarbonsäurelösung mit vermindertem Stickstoffgehalt wird in Stufe e) Wasser abdestilliert, z.B. unter einem Druck von 150 bis 950 mbar bis zu einer Temperatur von 130 bis 180°C unter Erhalt einer Schmelze von Dicarbonsäuren mit vermindertem Stickstoffgehalt.

In der nachfolgenden Stufe f) wird die Dicarbonsäureschmelze mit vermindertem Stickstoffgehalt auf eine Temperatur von 200 bis 240°C, insbesondere von 210 bis 230°C erhitzt. Vorteilhaft hält man im Zeitraum von 1 bis 3 Std. das Dicarbonsäuregemisch auf der angegebenen Temperatur.

In der anschließenden Stufe g) werden Dicarbonsäuren aus dem Dicarbonsäuregemisch durch Destillation gewonnen. Vorteilhaft führt man die Destillation unter vermindertem Druck z.B. von 5 bis 20 mbar bei einer Temperatur von 180 bis 200°C durch.

Das so erhaltene Dicarbonsäuregemisch zeichnet sich durch einen sehr niedrigen Gehalt von Stickstoff aus und eignet sich zur Herstellung von Estern mit Polyolen mit niedriger Farbzahl.

Das Verfahren nach der Erfindung wird in folgenden Beispielen veranschaulicht.

### Beispiel 1

Kupfer- und Vanadium-Rückgewinnung durch Ionentausch nach Schwermetallnitratzersetzung sowie Dicarbonsäuredestillation nach vorhergehender Zerstörung organischer Nitrokörper in Abwesenheit von Kupfer und Vanadium.

1 kg geschmolzenes Dicarbonsäuregemisch folgender Zusammensetzung, das durch Abdestillieren der Salpetersäure aus der Mutterlauge des Oxidationsprodukts von Cyclohexanol mit Salpetersäure und Abtrennung der Adipinsäure erhalten wird,

| | |
|---|---|
| Dicarbonsäuren *) | 95,6 % |
| freie Salpetersäure | 0,2 % |
| Nitrat | 2,8 % |
| Kupfer | 1,2 % |
| Vanadium | 0,14 % |
| Eisen | 0,005 % |

| | |
|---|---|
| *) incl. Anhydride, Imide und Nitrokörper (wie Pikrinsäure usf.) | |

wird unter Rühren für 15 Min. auf 160°C gehalten, wobei es zur Entwicklung eines aus NO₂, NO, N₂O und N₂ sowie CO und CO₂ bestehenden Gases kommt. Es verbleiben 0,91 kg Dicarbonsäuregemisch mit einem Nitratgehalt < 0,05 %, das zur Herstellung einer 60 %igen Lösung in Wasser gegossen wird. Die Lösung wird unter Rühren über 45 Min. auf 80°C gehalten und dann über einen geeigneten Kationenaustauscher gegeben. Die mit einem Wirkungsgrad von > 99 % von den Schwermetallionen befreite Lösung wird entwässert und die dabei anfallende Schmelze zur Zerstörung restlicher Oxalsäure und organischer Nitroverbindungen über 2 h auf 230°C erhitzt und dann bei 8 mbar in einem Sambay mit Manteltemperaturen von 180 bis 200°C der Destillation unterworfen. Das mit einer Destillationsausbeute von 85 % anfallende Dicarbonsäuregemisch (0,75 kg) hat einen N-Gehalt von 0,06 % und eine Polyetherolfarbzahl von 245 APHA.

Nach dem Verdrängen restlicher Dicarbonsäure aus dem Ionentauscher und anschließender Wäsche mit Wasser wird der Ionentauscher mit Salpetersäure eluiert. Das Eluat wird in einem Oxidationstest zur Oxidation von Cyclohexanol zu Adipinsäure eingesetzt. Die erhaltene Adipinsäure hat eine Farbzahl von 1,5 APHA.

### Vergleichsbeisplel 1

1 kg Schmelze von Dicarbonsäuregemisch der in Beispiel 1 beschriebenen Zusammensetzung wird bei 950 mbar über 3 h "bis zum Ende der anfangs stürmischen Gasentwicklung" auf 160-170°C gehalten. Das dunkelbraune Dicarbonsäuregemisch wird dann in Form einer 60 %igen wäßrigen Lösung über einen geeigneten Kationentauscher gegeben. Nach der Entwässerung der schwermetallfreien Lösung erfolgt unter den in Beispiel 1 beschriebenen Bedingungen eine Sambaydestillation mit einer Ausbeute von 78 %. N-Gehalt des Dicarbonsäuregemischs: 0,27 %; PEOL-Test: 1900 APHA.

Das unter vergleichbaren Bedingungen erhaltene Eluat ergab für die im Oxidationstest erzeugte Adipinsäure eine Farbzahl von 14,6 APHA. Anzumerken ist, daß es nur sehr schwer gelingt, den Ionentauscher wieder frei von braunen Belägen zu waschen.

### Vergleichsbeispiel 2

1 kg Schmelze von Dicarbonsäuregemisch der in Beispiel 1 beschriebenen Zusammensetzung wird bei 950 mbar über 3 h "bis zum Ende der anfangs stürmischen Gasentwicklung" auf 160 bis 170°C gehalten. Anschließend wird unter den in Beispiel 1 angegebenen Bedingungen eine Sambaydestillation durchgeführt, die wegen der Anwesenheit von Kupfer und Vanadium lediglich eine Ausbeute von 52 % zuläßt. Dicarbonsäuredaten: N-Gehalt 0,34 % PEOL-Test: 2600 APHA.

### Beispiel 2

### Eisenausschleusung mit und ohne vorhergehende Schwermetallnitratzersetzung

1 kg Dicarbonsäureschmelze der in Beispiel 1 angegebenen Zusammensetzung, jedoch mit einem Eisengehalt von 0,1 %, wird wie in Beispiel 1 beschrieben der thermischen Schwermetallnitratzersetzung bei 160°C unterworfen und in Form einer 60 %igen wäßrigen Lösung über den Ionentauscher gegeben. Man bricht die Zufuhr der Dicarbonsäurelösung ab, sobald im Austrag Vanadium/Kupfer nachzuweisen ist. Die AAS-Analyse zeigt, daß 70 % des Eisens mit der Dicarbonsäurelösung ausgeschleust werden konnten.

Wiederholt man den Versuch ohne Schwermetallnitratzersetzung, so stellt man fest, daß das gesamte Eisen vom Ionentauscher festgehalten wurde.

## Patentansprüche

1. Verfahren zur Aufarbeitung von Mutterlaugen, die bei der Herstellung von Adipinsäure durch Oxidation von Cyclohexanol oder Cyclohexanon oder deren Gemische mit Salpetersäure in Gegenwart von Kupfer- und Vanadiumkatalysatoren nach Abtrennung der Adipinsäure erhalten wurden, welches folgende Schritte umfasst
a) Abdestillieren der Salpetersäure als Azeotrop mit Wasser unter vermindertem Druck unter Erhalt einer Kupfer- und Vanadiumionen enthaltenden Dicarbonsäureschmelze,
b) Erhitzen der Kupfer- und Vanadiumionen enthaltenden Dicarbonsäureschmelze auf eine Temperatur von 130 bis 180°C unter Zersetzung von Stickstoffverbindungen und Oxalsäure und Erhalt einer Kupfer- und Vanadiumionen enthaltenden Dicarbonsäureschmelze mit vermindertem Stickstoffgehalt,
c) Lösen der Kupfer- und Vanadiumionen enthaltenden Dicarbonsäureschmelze mit vermindertem Stickstoffgehalt in Wasser unter Bildung einer wäßrigen Lösung, enthaltend Dicarbonsäuren mit vermindertem Stickstoffgehalt und Kupfer- und Vanadiumionen,
d) in Berührung bringen der Lösung enthaltend Dicarbonsäuren mit vermindertem Stickstoffgehalt und Kupfer- und Vanadiumionen mit einem Kationenaustauscher unter Bindung der Kupfer- und Vanadiumionen unter Erhalt einer im wesentlichen kupfer- und vanadiumfreien Dicarbonsäurelösung,
e) Abdestillieren von Wasser aus der im wesentlichen kupfer- und vanadiumionenfreien Dicarbonsäurelösung unter Erhalt einer Schmelze von Dicarbonsäuren,
f) Erhitzen der Schmelze von Dicarbonsäuren auf eine Temperatur von 200 bis 240°C und
g) Gewinnung von Dicarbonsäuregemisch durch Destillation.

2. Verfahren nach Anspruch 1, dadurch gekennzeichnet, daß man die Dicarbonsäureschmelze in Stufe b) für einen Zeitraum von 5 bis 60 Min. erhitzt.

3. Verfahren nach den Ansprüchen 1 und 2, dadurch gekennzeichnet, daß man in der Stufe f) die Dicarbonsäureschmelze für einen Zeitraum von 1 bis 3 Stunden erhitzt.

## Claims

1. A process for working up mother liquors resulting from the preparation of adipic acid by oxidation of cyclohexanol or cyclohexanone or mixtures thereof with nitric acid in the presence of copper and vanadium catalysts after removal of the adipic acid, which comprises the following steps
a) removal of the nitric acid by azeotropic distillation with water under reduced pressure to result in a dicarboxylic acid melt containing copper and vanadium ions,
b) heating said melt at from 130 to 180°C to decompose nitrogen compounds and oxalic acid and result in a dicarboxylic acid melt containing copper and vanadium ions and having a reduced nitrogen content,
c) dissolving said melt in water to form an aqueous solution containing dicarboxylic acids with a reduced nitrogen content and copper and vanadium ions,
d) contacting said aqueous solution with a cation exchanger to bind the copper and vanadium ions and result in an essentially copper- and vanadium-free dicarboxylic acid solution,
e) removal of water by distillation from said solution to result in a melt of dicarboxylic acids,
f) heating said melt at from 200 to 240°C and
g) obtaining a mixture of dicarboxylic acids by distillation.

2. A process as claimed in claim 1, wherein the dicarboxylic acid melt is heated in stage b) for from 5 to 60 min.

3. A process as claimed in claim 1 or 2, wherein the dicarboxylic acid melt is heated in stage f) for from 1 to 3 hours.

## Revendications

1. Procédé de traitement de liqueurs mères qui ont été obtenues au cours de la préparation d'acide adipique par oxydation de cyclohexanol, de cyclohexanone ou de leurs mélanges avec de l'acide nitrique en présence de catalyseurs au cuivre et au vanadium, après la séparation de l'acide adipique, comprenant les étapes suivantes:
a) Elimination par distillation de l'acide nitrique sous forme d'azéotrope avec de l'eau, sous pression réduite, avec obtention d'une masse fondue d'acides dicarboxyliques qui contient des ions cuivre et vanadium;
b) Chauffage de la masse fondue d'acides dicarboxyliques contenant des ions cuivre et vanadium à une température de 130 à 180°C, avec décomposition des composés azotés et de l'acide oxalique et obtention d'une masse fondue d'acides dicarboxyliques contenant des ions cuivre et vanadium et ayant une teneur réduite en azote;
c) Dissolution dans l'eau de la masse fondue d'acides dicarboxyliques contenant des ions cuivre et vanadium et ayant une teneur réduite en azote, avec formation d'une solution aqueuse contenant des acides dicarboxyliques à teneur réduite en azote et des ions cuivre et vanadium;
d) Mise en contact, avec un échangeur de cations, de la solution contenant des acides dicarboxyliques a teneur réduite en azote et des ions cuivre et vanadium, avec fixation des ions cuivre et vanadium, pour obtenir une solution d'acides dicarboxyliques pratiquement dépourvue de cuivre et de vanadium;
e) Elimination d'eau par distillation à partir de la solution d'acides dicarboxyliques pratiquement dépourvue d'ions cuivre et vanadium, pour obtenir une masse fondue d'acides dicarboxyliques:
f) Chauffage de la masse fondue d'acides dicarboxyliques à une température de 200 à 240°C; et
g) Récupération d'un mélange d'acides dicarboxyliques par distillation.

2. Procédé selon la revendication 1, caractérisé en ce que, dans l'étape b), on chauffe la masse fondue d'acides dicarboxyliques pendant une période de 5 à 60 mn.

3. Procéde selon la revendication 1 ou 2, caractérisé en ce que, dans l'étape f), on chauffe la masse fondue d'acides dicarboxyliques pendant une période de 1 à 3 h.
